Europäisches Patentamt

European Patent Office

Office européen des brevets

(11) Publication number: **0 197 206**
**A2**

## (12) EUROPEAN PATENT APPLICATION

(21) Application number: **85202137.7**

(22) Date of filing: **24.12.85**

(51) Int. Cl.⁴: **A 45 D 40/00**

(30) Priority: **04.04.85 IT 2132385 U**

(43) Date of publication of application:
**15.10.86 Bulletin 86/42**

(84) Designated Contracting States:
**AT BE CH DE FR GB IT LI LU NL SE**

(71) Applicant: **INTERCOS ITALIANA S.p.A.**
**Via Buonarroti, 66**
**I-20064 Gorgonzola (Milano)(IT)**

(72) Inventor: **Ferrari, Dario**
**Corso Venezia, 10**
**I-20121 Milano(IT)**

(74) Representative: **Mittler, Enrico et al,**
**c/o Marchi & Mittler s.r.l. Viale Lombardia, 20**
**I-20131 Milano(IT)**

(54) Article for the presentation of a pasty cosmetic product and the related production process.

(57) On a supporting stratum (1) are impressed depictions (2) formed with a pasty cosmetic product. For the production of said product a process is used which calls for preparation of a dry part and a fatty part appropriately mixed together and with a solvent in such a manner as to create a pasty cosmetic product of suitable density for application to and subsequent retention on the supporting stratum.

## Fig.1

EP 0 197 206 A2

"Article for the presentation of a pasty cosmetic product and the related production process"

\* \* \* \* \*

The present invention relates to an article for the presentation of a pasty cosmetic product and the related production process.

There are many pasty cosmetic products. Among them are to be listed eyeshadows, rouges and still others.

Problems connected with the presentation of the product in the form of small samples, whether to the public or to the retailer, are known.

The object of the present invention is to accomplish an article which makes it possible to present in a simple, effective and attractive manner samples of pasty cosmetic products.

In accordance with the invention said object is achieved by an article characterized in that it is made up of a supporting stratum bearing impressed depictions formed with the cosmetic product to be presented.

In other words, on a support made of paper or other material are reproduced in various ways inscriptions or illustrations using the cosmetic product as ink.

Given the characteristics of the cosmetic product, the user may remove the product from the supporting stratum, testing with the finger or other member the colour, consistency and so on of the cosmetic product.

As made, the article in accordance with the invention lends itself perfectly to the presentation of the cosmetic product to the public or to a retailer, and, for example, to inclusion in periodic publications as an advertising sample.

It should be noted that in this manner the cosmetic product does not need an individual containing envelope but is merely applied as an inscription or drawing on its support.

To accomplish the article according to the invention a process based on preparation of a pasty cosmetic product of a density such as to allow its application and then, after drying and hardening, its retention on the suporting layer is provided.

More precisely, the process according to the invention calls for separate preparation of a dry moiety and a fatty moiety. The dry moiety consists of a cosmetic powder including various pigments, silicates, clays, excipients and optionally silicas, all the ingredients being generally mixed coarsely to then be subjected to grinding and screening. The fatty moiety is made by mixing various fatty ingredients (e.g. lanolin and its derivatives, fatty esters, fatty acids, glycerol and its derivatives, mono-di-tri-glycerides and their derivatives, succinates and sulfo-derivatives, silicon and siloxane compounds, paraffin and mineral oils, chelating agents and preserving agents) at a temperature such as to dissolve the ingredient which melts at the highest temperature of the ingredients included in the mixture, until a homogeneous mixture is obtained.

The two moieties thus prepared are then mixed until a homogeneous fatty powder is obtained, to which is then added a solvent such as water or a mixture of water and another substance such as acetone, ethyl alcohol or isopropyl alcohol, or methylene chloride, silicon and siloxane compounds, or ethyl diglycol and its derivatives in appropriate quantity.

Alternatively, the fatty moiety is mixed with a waterless solvent, e.g. methylene chloride, silicon or siloxane compounds, or with water, e.g. water and acetone or water and isopropylic alcohol, until a solution in a biphase system or an emulsion in a triphase system is obtained respectively. Then the dry moiety is added.

In either case the type and quantity of solvent are selected in such a manner as to originate a homogeneous pasty product sufficiently fluid to be applied to the supporting stratum but also sufficiently dense to adhere to and be retained on the support.

The cosmetic product may be applied to the supporting stratum in various known ways, e.g. by serigraphy, pressing, and so on. The applied product is then allowed to dry and harden at surrounding temperature.

A practical embodiment of the article according to the invention is illustrated for greater clarity in the annexed drawings wherein:-

Fig. 1 shows a top view of an article in accordance with the invention;

Fig. 2 shows said articly in a sectional view along plane of cut II-II of Fig. 1.

The article illustrated as an example in the drawings consists of a supporting stratum 1, e.g. of paper, on which are applied inscriptions and/or illustrations 2 formed with pasty cosmetic products such as eyeshadow, rouge and so on.

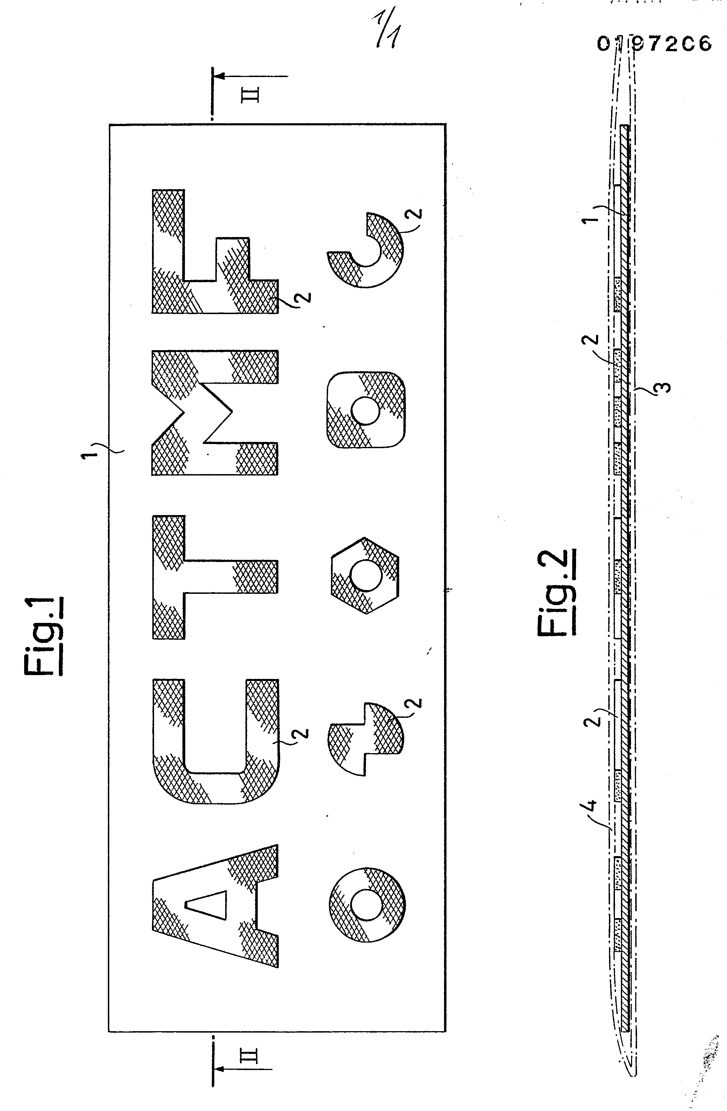

Even though not strictly necessary, in order to ensure appropriate preservation of the article it may be enclosed in a protective envelope (shown in broken lines in Fig. 2), formed of a base 3 and a transparent cover 4.

When being presented, a small quantity of the product may be removed with the finger from the inscription or drawing 2 in order to test the colour, consistency, perfume, etcetera thereof.

- 1 -

0197206

CLAIMS

1. Article for the presentation of a pasty cosmetic product characterized in that it is made up basically of a supporting stratum bearing impressed depictions formed with the cosmetic product to be presented.

2. Article in accordance with claim 1 characterized in that said depictions are impressed by serigraphy.

3. Article in accordance with claim 1 characterized in that said depictions are impressed by stamping.

4. Article in accordance with claim 1 characterized in that said supporting stratum is made of paper.

5. Article in accordance with claim 1 characterized in that it is equipped with a protective envelope having a transparent cover.

6. Production process for an article in accordance with the above claims characterized in that it comprises the following steps:-

a. preparation of a dry moiety consisting of a cosmetic powder,

b. preparation of a fatty moiety including various fatty ingredients mixed together,

c. mixing of the dry moiety with the fatty moiety until a homogeneous fatty powder is obtained, and d. addition of a solvent to said fatty powder until a pasty cosmetic product suitable for application to said supporting stratum is obtained.

7.   Process in accordance with claim 6 characterized in that said cosmetic powder includes various pigments, silicates, clays, possibliy silicas, and excipients.

8.   Process in accordance with claim 6 characterized in that said fatty ingredients are selected from among lanolin and its derivatives, fatty esters, fatty alcohols, glycerol and its esters, mono-di-tri-glycerides and their derivatives, succinates and sulfo-derivatives, silicon and siloxane compounds, paraffin and mineral oils, chelating agents, and preserving agents.

9.   Process in accordance with claim 6 characterized in that said solvent is selected from among water and mixtures of water and other substances such as acetone, ethyl alcohol and isopropyl alcohol, methylene chloride, silicon and siloxane compounds, and ethyldiglycol and its derivatives.

10.   Process in accordance with claim 6 characterized in that the pasty cosmetic product is applied to the supporting stratum by serigraphy.

11.   Process in accordance with claim 6 characterized in that the pasty cosmetic product is applied to the supporting stratum by stamping.

12.   Process for the production of an article in accordance with claims 1-5 characterized in that it comprises the following steps:-

a.   preparation of a dry moiety consisting of a cosmetic powder,

b.    preparation of a fatty moiety including various fatty ingredients mixed together,

c.    mixing of the fatty moiety with a solvent, and d.    addition of the dry moiety to obtain a pasty cosmetic product suitable for application to said supporting stratum.

13.    Process in accordance with claim 12 characterized in that said cosmetic powder includes various pigments, silicates, clays, optionally silicas, and excipients.

14.    Process in accordance with claim 12 characterized in that said fatty ingredients are selected from among lanolin and its derivatives, fatty esters, fatty alcohols, glycerol and its esters, mono-di-tri-glycerides and their derivatives, succinates and sulfo-derivatives, silicon and siloxane compounds, paraffins and mineral oils, chelating agents, and preserving agents.

15.    Process in accordance with claim 12 characterized in that the fatty moiety is mixed with a waterless solvent until a solution in a biphase system is obtained.

16.    Process in accordance with claim 15 charaterized in that said solvent is selected from among methylene chloride, silicon, and siloxane compounds.

17.    Process in accordance with claim 12 characterized in that the fatty moiety is mixed with a water-containing solvent until an emulsion in a triphase system is obtained.

18.    Process in accordance with claim 17 characterized in that said solvent consists of a mixture of water and another substance such as acetone or isopropyl alcohol.

19.    Process in accordance with claim 12 characterized in that the pasty cosmetic product is applied to the supporting stratum by serigraphy.

20.    Process in accordance with claim 12 characterized in that the pasty cosmetic product is applied to the supporting stratum by pressing.

## Fig.1

## Fig.2